# EUROPÄISCHE PATENTSCHRIFT

(11) **EP 2 853 247 B1**
(45) Veröffentlichungstag und Bekanntmachung des Hinweises auf die Patenterteilung: **27.07.2016**
(21) Anmeldenummer: 14003135.2
(22) Anmeldetag: 10.09.2014
(51) Int. Cl.: A61F 9/009

(54) **Patienteninterface für ophthalmologische, optische Therapie- und Diagnoseeinrichtung**
Patient inteface of ophthalmic and optic diagnosis and therapy device
Inteface patient pour un dispositif ophtalmique, optique de traitement ou diagnose

(30) Priorität: 26.09.2013 CH 16732013
(43) Veröffentlichungstag der Anmeldung: 01.04.2015
(73) Patentinhaber: Ziemer Ophthalmic Systems AG, 2562 Port (CH)
(72) Erfinder: Rathjen, Christian, 28197 Bremen (DE); Studer, Thomas, 2000 Neuchâtel (CH)
(74) Vertreter: Rentsch Partner AG

(56) Entgegenhaltungen:
- US-A1- 2008 071 254
- US-A1- 2012 283 708

## Beschreibung

### Technisches Gebiet

Die vorliegende Erfindung betrifft ein Patienteninterface zur Kopplung eines ophthalmologischen Applikationskopfes zur Anwendung von optischer Strahlung einer Strahlquelle an ein Patientenauge sowie ein Zwischenelement zur Anordnung zwischen einem Patienteninterface und dem ophthalmologischen Applikationskopf. Die Erfindung betrifft ferner einen zur Verwendung in Verbindung mit einem Patienteninterface ausgelegten ophthalmologischen Applikationskopf zur Anwendung von optischer Strahlung einer Strahlquelle. Die Erfindung betrifft weiterhin ein Verfahren zur Kopplung des ophthalmologischen Applikationskopfes an ein Patientenauge.

### Stand der Technik

Zur Behandlung und/oder Diagnose von Augengewebe ist der Einsatz von Lasern bekannt. Entsprechende Vorrichtungen weisen beispielsweise ein Basisgerät mit einer Laser-Lichtquelle zur Erzeugung von Laserpulsen, beispielsweise Femtosekundenlaserpulse, sowie einen Applikationskopf mit einem Projektionsobjektiv auf, der zur Behandlung mit dem Patientenauge gekoppelt wird. Der Applikationskopf kann, beispielsweise über einen Gelenkarm, beweglich mit dem Basisgerät verbunden sein, wobei der Gelenkarm zugleich der optischen Strahlführung von der Laser-Lichtquelle zum Applikationskopf dienen kann. Eine entsprechende Anordnung ist beispielsweise in der EP 1 731 120 offenbart. Des Weiteren gibt es Geräte bei denen der Applikationskopf im Basisgerät integriert ist oder bei denen andere Vorrichtungsanordnungen vorgesehen sind.

Die mechanische und optische Kopplung des Applikationskopfes an das Patientenauge, beispielsweise an die Hornhaut und/oder Sklera des Patientenauges, erfolgt über ein Patienteninterface, wobei das Patienteninterface einen transparenten Kontaktkörper umfassen kann, durch welchen die aus dem Projektionsobjektiv austretenden Laserpulse geleitet werden und der durch mechanischen Kontakt mit der Hornhaut diese bezüglich des Patienteninterface und des Projektionsobjektives fixiert. Alternativ zur Kopplung mittels eines Kontaktkörpers kann eine Flüssigkeitskopplung vorgesehen werden, wobei sich zwischen Hornhaut und Projektionsobjektiv eine Koppelflüssigkeit, beispielsweise physiologische Kochsalzlösung befindet. Entsprechende Patienteninterfaces sind beispielsweise aus WO2012031277 bekannt. Die Kopplung des Patienteninterface an das Patientenauge kann mittels Vakuum und einem auf die Hornhaut aufgesetzten Saugring erfolgen. Die Meisten Saugringe haben zwei Dichtlippen. Die Lippen können auf der Sklera, der Sklera und der Hornhaut oder nur auf der Hornhaut angebracht sein. Weiterhin gibt es Varianten, die nur einen Ring besitzen und über dem ganzen Auge ein Vakuum erzeugen, oder Varianten, die aus mehreren Saugkammern/Saugnäpfen bestehen. Der Saugring ist die gebräuchlichste Methode der Befestigung, es gibt jedoch auch andere bekannte Lösungen. Die Kopplung des Patienteninterface an den Applikationskopf erfolgt bei bekannten Systemen beispielsweise mittels Schraubverbindung, Bajonettverschlüssen oder Vakuumkupplungen.

US 2008/287927 beschreibt eine Schutzvorrichtung für die ophthalmologische Laserbehandlung mittels einer Schutzfolie zum Schutz des Auges vor direktem Kontakt mit einem Referenzkörper, der zwischen dem Applikationskopf und dem Auge angeordnet ist. Die Schutzvorrichtung umfasst einen Saugring zur Befestigung am Auge und ist eingerichtet, die Schutzfolie aufzunehmen, beispielsweise mittels eines ringförmigen Trägerrahmens. Der Saugring ist mit Kopplungsmitteln versehen, welche der Befestigung am Applikationskopf dienen. Gemäss US 2008/287927 sind die Kopplungsmittel als Schraubverschluss, Bajonettverschluss oder Schnappverschluss ausgestaltet.

US 2012/0283708 beschreibt ein ophthalmologisches Patienteninterface, das an einen Applikationskopf eines Lasersystems ankoppelbar ist und eine aufs Auge aufsetzbare Kontaktlinse aufweist. US 2012/0283708 beschreibt ein an der Kontaktlinse angebrachtes flexibles Verbindungselement, das beim und während dem Andocken eine rotationsmässige und/oder transversale Bewegung der Kontaktlinse bezüglich des Applikationskopfs ermöglicht. Ohne weitere Beschreibung dazu erwähnt das Dokument einen "Flex-and-Lock" Mechanismus, mit dem eine weitere Bewegung der Kontaktlinse bezüglich des Applikationskopfs unterbunden werden kann.

Für Patienteninterfaces, die bei ophthalmologische Laseranwendungen eingesetzt werden, ergeben sich eine Reihe von Anforderungen und Randbedingungen, welche die Übertragung zahlreicher aus anderen Anwendungsgebieten bekannten Konstruktionen verunmöglichen bzw. als aus praktischer Sicht impraktikabel erscheinen lassen. Wesentliche Beschränkungen ergeben sich aus der bestehenden Nähe der Vorrichtungen zum Patientenauge und insbesondere dem direkten physischen Kontakt des Patienteninterface mit dem Patientenauge. Neben hohen Sicherheitsanforderungen ergibt sich hieraus, dass zumindest das Patienteninterface leicht handhabbar und sicher zu befestigen ausgestaltet ist.

Obgleich die Platzverhältnisse in der Umgebung des Einsatzortes am Patientenauge eng und gedrängt sind, sollte das Patientenauge für den Benutzer während der Behandlung zugänglich und sichtbar sein. Patienteninterface und übrige Komponenten sollten daher die Zugänglichkeit und Sicht möglichst gering beeinträchtigen.

Aufgrund der erheblichen medizinischen Risiken bei einer Fehlleitung des Laserstrahls, die zu unerwünschter Beeinträchtigung und gar Zerstörung von Augengewebe führen kann, sind Komponenten, die im Bereich des Strahlengangs vom Lichtprojektor zum Patientenauge angeordnet werden, korrekt zu positionieren und auszurichten sowie sicher miteinander zu koppeln bzw. zu verbinden.

### Darstellung der Erfindung

Es ist eine Aufgabe der vorliegenden Erfindung, Vorrichtungen, insbesondere ein Patienteninterface, für ophthalmologische Laseranwendungen sowie mit diesem Patienteninterface interagierende Vorrichtungen bereitzustellen, welche zumindest einige Nachteile des Stands der Technik nicht aufweisen. Es ist insbesondere eine Aufgabe der vorliegenden Erfindung, ein Patienteninterface, ein Zwischenelement und einen Applikationskopf zur ophthalmologischen Anwendung von optischer Strahlung einer Strahlquelle vorzuschlagen, welche wenigstens gewisse der eingehend erläuterten Anforderungen an die Kopplung eines Patienteninterface an einen Applikationskopf hinsichtlich einfacher und sicherer Verbindung ganz oder teilweise erfüllen.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Ein Patienteninterface zur Kopplung eines ophthalmologischen Applikationskopfes zur Anwendung von optischer Strahlung einer Strahlquelle an ein Patientenauge umfasst:
- eine patientenseitige Interfacestruktur, die zur Kopplung an das Patientenauge ausgestaltet ist, und
- eine quellseitige Interfacestruktur, welche dazu ausgelegt ist, das Patienteninterface starr an den Applikationskopf oder an ein zur Anordnung zwischen Applikationskopf und Patienteninterface vorgesehenes Zwischenelement zu koppeln.

Die oben genannten Ziele werden durch die vorliegende Erfindung insbesondere dadurch erreicht, dass die quellseitige Interfacestruktur einen ersten quellseitigen Patienteninterfacekoppler und einen zusätzlichen zweiten quellseitigen Patienteninterfacekoppler umfasst. Der erste quellseitige Patienteninterfacekoppler ist dazu ausgelegt, durch Herstellen einer Kopplung mit einem ersten Patienteninterfacekoppler-Gegenstück des Applikationskopfes oder des Zwischenelements die Beweglichkeit des Patienteninterface bezüglich des Applikationskopfes oder des Zwischenelements einzuschränken. Der zweite quellseitige Patienteninterfacekoppler ist dazu ausgelegt, durch Herstellen einer Kopplung mit einem zweiten Patienteninterfacekoppler-Gegenstück des Applikationskopfes oder des Zwischenelements das Patienteninterface bei bestehender Kopplung des ersten quellseitigen Patienteninterfacekopplers mit dem ersten Patienteninterfacekoppler-Gegenstück starr an den Applikationskopf oder das Zwischenelement zu koppeln. Der erste quellseitige Patienteninterfacekoppler weist somit den Vorteil auf, dass er dazu ausgelegt, die Beweglichkeit des Patienteninterface bezüglich des Applikationskopfes oder des Zwischenelements durch Herstellen einer Kopplung mit dem ersten Patienteninterfacekoppler-Gegenstück des Applikationskopfes oder des Zwischenelements einzuschränken, bevor eine Kopplung des zweiten quellseitigen Patienteninterfacekopplers mit einem zweiten Patienteninterfacekoppler-Gegenstück des Applikationskopfes oder des Zwischenelements vorgenommen wird, das heisst ohne dass für die Einschränkung der Beweglichkeit des Patienteninterface bezüglich des Applikationskopfes oder des Zwischenelements eine Kopplung des zweiten quellseitigen Patienteninterfacekopplers mit dem zweiten Patienteninterfacekoppler-Gegenstück des Applikationskopfes oder des Zwischenelements erforderlich ist.

Die Begriffe "quellseitig" und "patientenseitig" in Bezug auf ein Element bezeichnen, dass das Element in der betriebsgemässen Ausrichtung dem Applikationskopf bzw. dem Patientenauge zugewandt und im betriebsgemässen Zustand direkt oder indirekt mit diesem verbunden ist.

Eine Einschränkung der Beweglichkeit bedeutet, dass die Zahl der mechanischen Freiheitsgrade zur Bewegung reduziert wird, aber zumindest ein Freiheitsgrad weiterhin vorhanden ist. Eine starre Kopplung bedeutet die Aufhebung sämtlicher Freiheitsgrade, wodurch sich die starr gekoppelten Elemente im Wesentlichen wie ein gemeinsamer Körper verhalten.

In einer Ausführungsvariante sind genau zwei Kopplungen bzw. Koppelstellen vorgesehen, welche so angeordnet sind, dass bei korrekt erstellter erster und zweiter Kopplung keine Relativbewegungen des Patienteninterface gegenüber dem Applikationskopf respektive dem Zwischenelement möglich sind, das Patienteninterface jedoch seine Position und/oder Orientierung gegenüber dem Applikationskopf oder dem Zwischenelement ändert, wenn mindesten eine der Kopplungen nicht mehr korrekt gegeben ist, beispielsweise dadurch, dass das Patienteninterface schwerkraftbedingt vom Applikationskopf bzw. dem ggf. vorhandenen Zwischenelement abfällt und in einer Variante mit entsprechender Ausgestaltung in der veränderten Position den Eintritt des Laserstrahls in das Patientenauge blockiert. Dem gegenüber birgt eine Kopplung von Patienteninterface und Applikationskopf oder Zwischenelement über eine grössere Zahl von drei und mehr Koppelpunkten die Gefahr, dass sich beispielsweise eine von drei Kopplungen während des Betriebs löst, wobei die verbleibenden Kopplungen das Patienteninterface weiterhin am Applikationskopf oder dem Zwischenelement halten, aber nicht mehr hinreichend starr fixieren, so dass Relativbewegungen möglich werden, ohne dass dies vom Benutzer bemerkt wird und/oder der Eintritt des Laserstrahls in das Patientenauge verhindert wird. Dabei ist anzumerken, dass die visuelle Überprüfung einer Kopplung insbesondere bei mehr als zwei Kopplungspunkten schwer bis gar nicht möglich ist, wenn sich das Patienteninterface am Auge befindet.

Im montieren Zustand des Patienteninterface ermöglicht eine starre Kopplung mit dem Applikationskopf oder dem gegebenenfalls vorhanden Zwischenelement eine korrekte Ausrichtung der Laserpulse auf einen Zielpunkt oder ein Zielgebiet im Patientenauge.

Der erste und der zweite quellseitige Patienteninterfacekoppler sind bevorzugt an einander gegenüberliegenden Seiten oder Wandungen des Patienteninterface angeordnet.

Das Patienteninterface gibt somit für die Montage an den Applikationskopf oder das ggf. vorhandene Zwischenelement das folgende Vorgehen vor: Ausgehend von einem ungekoppelten Ausgangszustand in dem das Patienteninterface nicht mit dem Applikationskopf bzw. dem ggf. vorhandenen Zwischenelement gekoppelt und von diesem separat ist, wird zunächst eine erste Kopplung durch Herstellung einer Kopplung des ersten quellseitigen Patienteninterfacekopplers mit dem ersten Patienteninterfacekoppler-Gegenstück herbeigeführt. Unter Beibehaltung dieser ersten Kopplung (und nur dann) kann das Patienteninterface durch eine Bewegung, z. B. durch eine Kipp- oder Schwenkbewegung bezüglich des Applikationskopfes bzw. des vorhandenen Zwischenelements bewegt werden, bis durch Herstellung einer Kopplung des zweiten quellseitigen Patienteninterfacekopplers mit dem zweiten Patienteninterfacekoppler-Gegenstück zusätzlich eine zweite Kopplung hergestellt ist.

Vorteilhaft sind das Patienteninterface sowie der Applikationskopf bzw. das ggf. vorhandene Zwischenelement derart gestaltet, dass die zur Herstellung der zweiten Kopplung erforderliche Bewegung des Patienteninterface mit einer (einzigen) Hand möglich ist.

Der erste und der zweite quellseitige Interface-Koppler sind für eine lösbare Kopplung, insbesondere eine zerstörungsfrei lösbare Kopplung, mit den jeweiligen Gegenstücken ausgelegt. Wie weiter unten dargestellt wird, können die Koppler für ein einfaches Lösen mit wenigen Handmanipulationen bei einhändiger Bedienung ausgestaltet werden.

Die einhändige Erstellung und Lösung der Kopplung des Patienteninterface mit dem Applikationskopf bzw. dem Zwischenelement ist insbesondere von Vorteil beim Einsatz von ophthalmologischen Lasersystemen, bei denen der Applikationskopf an einem Schwenkarm befestigt ist und mit der Hand gehalten respektive geführt wird. Dem Benutzer wird so ermöglicht, den Applikationskopf mit der einen Hand zu halten und mit der anderen die Kopplung mit respektive Loslösung von dem Patienteninterface und einem ggf. vorhandenen Zwischenelement mit wenigen einfachen Handgriffen vorzunehmen.

Das Patienteninterface ist vorteilhaft als Einweg-Produkt zur einmaligen Anwendung bei einer Augenbehandlung und anschliessender Entsorgung ausgeführt. Das Patienteninterface kann als einfaches oder Mehrkomponenten- Spritzgussteil aus Kunststoff ausgeführt sein. Zusätzlich oder alternativ können weitere Materialien wie Metall und/oder weitere Fertigungsverfahren, wie Tiefziehen oder spanende Bearbeitung oder 3D-Drucktechnik eingesetzt werden.

Das Patienteninterface kann schalenartig ausgebildet sein und zum Applikationskopf bzw. Zwischenelement hin im Wesentlichen eine konkave und zum Patienten hin im Wesentlichen eine konvexe Formgebung aufweisen, wobei die konkave Formgebung komplementär zu einer entsprechenden konvexen Formgebung des Applikationskopfes oder Zwischenelementes sein kann.

In einer beispielhaften Ausführungsform ist der erste quellseitige Patienteninterfacekoppler dazu ausgelegt ist, unter Beibehaltung mindestens eines Freiheitsgrades mit dem ersten Patienteninterfacekoppler-Gegenstück formschlüssig zu koppeln. Der Formschluss kann durch eine näherungsweise punktartige Kopplung erfolgen. So kann der erste quellseitige Patienteninterfacekoppler als Ausschnitt, Bohrung, Sackloch oder generell ein konkaves Element, z. B. eine Mulde, in einer Wandung des Patienteninterface ausgebildet sein. Das korrespondierende erste Patienteninterfacekoppler-Gegenstück kann in diesem Fall als konvexes Element, beispielsweise als Stift oder Noppen, an einer Wandung des Applikationskopfes oder Zwischenelementes vorgesehen sein. Die Anordnung ist derart, dass beim Ansetzen des Patienteninterface an den Applikationskopf oder das Zwischenelement das konvexe Element und das konkave Element bzw. die Bohrung oder der Ausschnitt in Eingriff gelangen und dadurch eine formschlüssige Verbindung bilden. Alternativ oder zusätzlich kann die Kopplung beispielsweise magnetisch oder durch Vakuum erfolgen. Ferner kann ein konvexes Element am Patienteninterface und ein korrespondierendes konkaves Element, ein Ausschnitt oder ein Bohrung am Applikationskopf oder Zwischenelement angeordnet sein. Je nach Gestalt, Abmessungen und Gewicht des Patienteninterface können der erste quellseitige Patienteninterfacekoppler und der korrespondierende erste Gegenkoppler jeweils auch durch mehrere Einzelelemente realisiert werden, beispielsweise zwei oder mehr Stifte oder Noppen und eine entsprechende Anzahl konkaver Elemente, Bohrungen oder Ausschnitte. Die Einzelelemente sind dabei vorteilhafterweise so anzuordnen, dass sie konstruktionsbedingt gleichzeitig eine Kopplung herstellen bzw. auflösen. Zu jedem Zeitpunkt sind damit entweder alle den ersten quellseitigen Patienteninterfacekoppler und den korrespondierenden ersten Gegenkoppler bildenden Elementpaare jeweils gemeinsam entweder im gekoppelten oder ungekoppelten Zustand.

In einer beispielhaften Ausführungsform ist der erste quellseitige Patienteninterfacekoppler dazu ausgelegt, durch Herstellen einer Kopplung mit dem ersten Patienteninterfacekoppler-Gegenstück eine Zwangsführung für das Patienteninterface bezüglich des Applikationskopfes oder des Zwischenelements herzustellen. Die Zwangsführung kann das Patienteninterface bezüglich des Applikationskopfes oder des Zwischenelementes soweit festlegen, dass für das Patienteninterface bei vorhandener Kopplung des ersten quellseitigen Patienteninterfacekopplers und des ersten Interface-Gegenkopplers nur eine Bewegung, beispielsweise eine Kipp- oder Schwenkbewegung, in eine Richtung möglich ist, die zur Herstellung einer Kopplung des zweiten quellseitigen Patienteninterfaces mit dem zweiten Patienteninterfacekoppler-Gegenstück führt. Eine derartige Zwangsführung kann dadurch realisiert werden, dass Innenwandungen der konkaven Form des Patienteninterface nach Herstellung der Kopplung zwischen dem ersten quellseitigen Patienteninterfacekoppler und dem ersten Interface-Gegenkoppler im Wesentlichen spielfrei an korrespondierende Aussenwandungen des Applikationskopfes anliegen und für diese eine Gleitführung bilden.

In einer beispielhaften Ausführungsform erfordert das Herstellen einer Kopplung des zweiten quellseitigen Patienteninterfacekopplers mit dem zweiten Patienteninterfacekoppler-Gegenstück, dass der erste quellseitige Patienteninterfacekoppler mit dem ersten Patienteninterfacekoppler-Gegenstück gekoppelt ist. Eine derartige Ausführungsform stellt sicher, dass eine Montage des Patienteninterface nur in der vorgesehenen und korrekten Weise möglich ist und im montierten Zustand das Patienteninterface bezüglich des Applikationskopfes oder des Zwischenelementes korrekt positioniert und orientiert ist. Vorteilhaft muss vom Benutzer nur der zweite Interfacekoppler überprüft werden.

In einer beispielhaften Ausführungsform ist der zweite quellseitige Patienteninterfacekoppler dazu ausgelegt, mit dem zweiten Patienteninterfacekoppler-Gegenstück mittels Schnapp- oder Rastverbindung zu koppeln. Hierzu kann das Patienteninterface als erster quellseitiger Patienteninterfacekoppler eine federnde Klinke und das erste Patienteninterfacekoppler-Gegenstück eine korrespondierende Gegenklinke aufweisen. Die Federklinke und die Gegenklinke können dabei derart angeordnet sein, dass sie in einer Endstellung des Patienteninterface relativ zum Applikationskopf oder Zwischenelement zwangsläufig in Eingriff geraten und so das Patienteninterface mit dem Applikationskopf oder dem Zwischenelement verriegeln oder verrasten. Die Bewegungs-Endposition des Patienteninterface entspricht dabei der Position und Orientierung des Patienteninterface relativ zum Applikationskopf oder Zwischenelement im betriebsbereiten Zustand. In einer beispielhaften Anordnung umfasst der zweite quellseitige Patienteninterfacekoppler dabei einen einhändig lösbaren Schnapphebel, beispielsweise eine Federklinke. Dieser kann federnd gestaltet sein und an seinem Ende eine Klinke zum Verrasten mit der Gegenklinke aufweisen. Ein einhändiges Lösen kann mittels einer Entriegelungstaste vorgesehen werden, welche Teil des Schnapphebels ist und mit welcher sich durch federndes Biegen der Klinke der Eingriff von Klinke und Gegenklinke aufheben lässt, beispielsweise mittels Fingeroder Daumendruck. Wird der zweite quellseitige Patienteninterfacekoppler, beispielsweise aus Steifigkeitsgründen, durch eine Kombination mehrerer einzelner Schnapphebel realisiert, können diese strukturell verbunden sein, um ein gemeinsames Aufheben des Schnapp- oder Rasteingriffs mit der Gegenklinke und damit ein Aufheben der Kopplung zu ermöglichen.

In einer alternativen Ausführungsform können die Anordnung von Federklinke und Gegenklinke vertauscht werden. In weiteren Ausführungsformen werden statt zusammenwirkenden Klinken eine Anordnung aus konvexen Elementen am Patienteninterface, beispielsweise Noppen, und korrespondierenden konkaven Elementen, Ausschnitten oder Bohrungen am Applikationskopf oder Zwischenelement verwendet, oder umgekehrt.

Schnapp- und Rastverbindungen sind insoweit besonders günstig, als das vollendete Herstellen der Kopplung, also das Einrasten oder Einschnappen, aufgrund der hierbei auftretenden Kraftdiskontinuität gut taktil feststellbar und ferner mit einem charakteristischen Schnapp- oder Klackgeräusch verbunden ist, was eine zusätzliche akustische Rückmeldung an den Benutzer darstellt.

Grundsätzlich können der zweite quellseitige Patienteninterfacekoppler und der korrespondierende zweite Gegenkoppler auch durch zwei oder mehr Paare von konstruktiven Einzelelementen realisiert werden. Dabei kann die Zahl der Einzelelemente für den ersten und den zweiten quellseitigen Patienteninterfacekoppler gleich oder verschieden sein. So sind beispielsweise Ausführungen möglich, in denen der erste quellseitige Patienteninterfacekoppler durch zwei Bohrungen gebildet werden, die zur Kopplung mit zwei korrespondierenden Stiften als erstem Gegenkoppler in Eingriff gelangen, während der zweite quellseitige Patienteninterfacekoppler durch eine einzelne, zu den Bohrungen symmetrisch angeordnete Federklinke gebildet wird.

In einer beispielhaften Ausführungsform ist das Patienteninterface mindestens teilweise elastisch. Beim Herstellen der Kopplung des zweiten quellseitigen Patienteninterfacekopplers mit dem zweiten Patienteninterfacekoppler-Gegenstück wird das Patienteninterface federnd gespannt. Eine federnde Spannung des Patienteninterface gewährleistet einen straffen und spielfreien Sitz des Patienteninterface auf dem Applikationskopf oder dem Zwischenelement unter Gewährleistung der korrekten Position und Orientierung. Eine mindestens teilweise Elastizität des Patienteninterface kann durch geeignete Formgebung des Patienteninterface sowie die Materialauswahl, beispielsweise bei Herstellung im Spritzgussverfahren, erfolgen. Bei einer solchen Ausgestaltung weist das Patienteninterface insgesamt die erforderliche Elastizität auf. Alternativ können auch spezielle federnde Elemente, z. B. Federn eingesetzt werden. Ferner kann eine Elastizität durch den ersten und/oder den zweiten quellseitigen Patienteninterfacekoppler realisiert werden. Hierzu werden diese so ausgelegt, dass sie im gekoppeltem Zustand, also bei bestehendem Eingriff mit dem korrespondierenden Gegenkoppler, unter Vorspannung stehen.

In einer beispielhaften Ausführungsform erfordert das Herstellen der Kopplung des ersten quellseitigen Patienteninterfacekopplers mit dem ersten Patienteninterfacekoppler-Gegenstück, dass der zweite quellseitige Patienteninterfacekoppler mit dem zweiten Patienteninterfacekoppler-Gegenstück nicht gekoppelt ist. Eine derartige Ausgestaltung stellt entsprechend sicher, dass - entsprechend der vorgesehenen Montagereihenfolge für eine korrekte Platzierung des Patienteninterface - immer zuerst die Verbindung des ersten quellseitigen Patienteninterfacekopplers mit seinem korrespondieren Gegenstück erfolgt und erst anschliessend die Verbindung des zweiten quellseitigen Patienteninterfacekopplers. Dieses Ziel lässt sich durch geeignete Anordnung vom ersten und zweiten quellseitigen Patienteninterfacekoppler sowie geeignete Ausgestaltung dieser Koppler erzielen, wie nachfolgend anhand von Ausführungsbeispielen dargestellt wird.

In einer beispielhaften Ausführungsform umfasst das Patienteninterface ein mit einer Hand haltbare Griffstruktur. In einer möglichen Ausführung ist diese Griffstruktur integral mit dem zweiten quellseitigen Patienteninterfacekoppler. Eine mit der Hand haltbare Griffstruktur ist günstig zur Feinpositionierung und Ausrichtung des Patienteninterface vor bzw. bei der Kopplung mit dem Applikationskopf oder dem Zwischenelement. Eine Griffstruktur erscheint insbesondere dann vorteilhaft, wenn - entsprechend einer möglichen Anwendung des Patienteninterface - zunächst das Patienteninterface an das Patientenauge gekoppelt wird und anschliessend die Verbindung mit dem Applikationskopf erfolgt. Zur Integration in den zweiten quellseitige Patienteninterfacekoppler kann ein federnder Schnapphebel, beispielsweise wie oben erläutert eine Entriegelungstaste eines Schnapphebels, als Griffstruktur ausgebildet sein.

In einer beispielhaften Ausführungsform umfasst die patientenseitige Interfacestruktur einen Saugring, welcher zum Herstellen einer starren Vakuumkopplung mit dem Patientenauge vorgesehen ist. Der Saugring ist zum Aufsetzen auf die Hornhaut des Patientenauges vorgesehen und kann eine doppelwandige Struktur mit einer inneren und einer zu dieser koaxialen und äquidistanten äusseren Wandung aufweisen, wobei der Freiraum zwischen der inneren Wandung und der äusseren Wandung als eine zum Patientenauge hin offene ringförmige Hohlkammer zur Ausbildung eines Unterdrucks oder Vakuums ausgestaltet ist. Hierzu kann der Saugring einen mit dem Zwischenraum fluidisch gekoppelten Anschluss, beispielsweise einen Stutzen, für die Verbindung mit einer Unterdruckoder Vakuumpumpe aufweisen. Bei einer derartigen Anordnung verläuft der Laserstrahl bei der Anwendung durch den von der inneren Wandung umgebenen Freiraum.

In einer beispielhaften Ausgestaltung ist das Patienteninterface dafür ausgelegt, teilweise mit einer optischen Koppelflüssigkeit, beispielsweise physiologische Kochsalzlösung, gefüllt zu werden. Die einzufüllende Menge der Koppelflüssigkeit wird dabei so bemessen, dass ein, nach Kopplung des Patienteninterface an den Applikationskopf oder das Zwischenelement, zwischen dem Patientenauge und dem Applikationskopf oder dem Zwischenelement bestehender Freiraum vollständig flüssigkeitsgefüllt ist. Das Befüllen mit Koppelflüssigkeit erfolgt bevorzugt nach der Kopplung des Patienteninterface an das Patientenauge und vor der Kopplung des Patienteninterface an den Applikationskopf oder das Zwischenelement. Statt einer Flüssigkeitsfüllung kann die Kopplung des Applikationskopfes an das Patientenauge auch mittels eines auf das Patientenauge aufgesetzten Kontaktkörpers erfolgen. Der Kontaktkörper kann Teil des Applikationskopfes oder des Patienteninterfaces sein. Zusätzlich können noch Membranen und Folien als Sterilbarriere eingesetzt werden.

Gemäss einem weiteren Aspekt wird ein Zwischenelement zur Anordnung zwischen einem Patienteninterface und einem ophthalmologischen Applikationskopf zur Anwendung von optischer Strahlung einer Strahlquelle bereitgestellt.

Das Zwischenelement ist zur Anordnung zwischen einem ophthalmologischen Applikationskopf zur Anwendung von optischer Strahlung einer Strahlquelle und einem offenbarungsgemässen Patienteninterface vorgesehen und umfasst:
- eine patientenseitige Zwischenelement-Interfacestruktur und
- eine quellseitige Zwischenelement-Interfacestruktur.

Die patientenseitige Zwischenelement-Interfacestruktur umfasst einen ersten patientenseitigen Zwischenelementkoppler und einen zusätzlichen zweiten patientenseitigen Zwischenelementkoppler. Der erste patientenseitige Zwischenelementkoppler bildet dabei ein erstes Patienteninterfacekoppler-Gegenstück und der zweite patientenseitige Zwischenelementkoppler bildet ein zweites Patienteninterfacekoppler-Gegenstück.

Der erste patientenseitige Zwischenelementkoppler ist dazu ausgelegt, durch Herstellen einer Kopplung mit dem ersten quellseitigen Patienteninterfacekoppler des Patienteninterface die Beweglichkeit des Patienteninterface bezüglich des Zwischenelements einzuschränken. Der zweite patientenseitige Zwischenelementkoppler ist dazu ausgelegt, durch Herstellen einer Kopplung mit dem zweiten quellseitigen Patienteninterfacekoppler des Patienteninterface bei bestehender Kopplung des ersten patientenseitigen Zwischenelementkopplers mit dem ersten quellseitigen Patienteninterfacekoppler das Patienteninterface starr an das Zwischenelement zu koppeln.

Das Zwischenelement ist zur Anordnung zwischen Patienteninterface und Applikationskopf vorgesehen, wobei sich im montierten Zustand eine Sandwich-Struktur ergibt. Das Zwischenelement kann - ebenso wie das Patienteninterface -schalenartig ausgebildet sein. Zum Applikationskopf hin weisst es dann eine im Wesentlichen konkave Formgebung auf, die komplementär zu einer im Wesentlichen konvexen Formgebung des Applikationskopfes ist. Zum Patienteninterface weisst ein solches Zwischenelement eine im Wesentlichen konvexe Formgebung auf, die komplementär zu einer im Wesentlichen konkaven Formgebung des Patienteninterface ist. In einer solchen Anordnung bedeckt oder umhüllt das Zwischenelement einen dem Patienten zugewandten Teil des Applikationskopfes wie eine Schutzhülle.

Der erste und zweite patientenseitige Zwischenelementkoppler sind zur Interaktion mit dem ersten und zweiten quellseitigen Patienteninterfacekoppler des Patienteninterface ausgestaltet. Wenn der erste quellseitige Patienteninterfacekoppler etwa als konkaves Element, beispielsweise ein Pin, Stift oder Noppen, gebildet wird, ist der erste patientenseitige Patienteninterfacekoppler entsprechend als konvexes Element, beispielsweise in Form einer Mulde, einer Bohrung oder eines Ausschnitts, gebildet, und umgekehrt. Entsprechendes gilt für den zweiten patientenseitigen Zwischenelement-Koppler.

Ein offenbarungsgemässes Zwischenelement kann einen optisch transparenten Trennkörper, beispielsweise in Form einer transparenten Folie aufweisen, der im montierten Zustand an das Austrittsfenster oder Projektionsobjektiv des Applikationskopfes anliegt.

Das optionale Zwischenelement trennt den Applikationskopf vom Patienteninterface und schützt den Applikationskopf beispielsweise vor Verschmutzungen. Wird entsprechend obiger Beschreibung eine optische Koppelflüssigkeit eingesetzt, schützt der optional vorhandene transparente Trennkörper, z.B. eine Folie oder Membran, ferner den Applikationskopf vor Kontakt mit der ggf. zusätzlich durch Keime und Gewebezellen verunreinigten Kontaktflüssigkeit. Alternativ kann das Zwischenelement auch einen Kontaktkörper beinhalten.

In einer beispielhaften Ausführungsform ist die quellseitige ZwischenelementInterfacestruktur dazu ausgelegt, das Zwischenelement starr an den Applikationskopf zu koppeln. Die quellseitige Zwischenelement-Interfacestruktur umfasst dabei einen ersten quellseitigen Zwischenelementkoppler und einen zweiten quellseitigen Zwischenelementkoppler. Der erste quellseitige Zwischenelementkoppler ist dazu ausgelegt, durch Herstellen einer Kopplung mit einem ersten Applikationskopfkoppler des Applikationskopfes die Beweglichkeit des Zwischenelements bezüglich des Applikationskopfes einzuschränken. Der zweite quellseitige Zwischenelementkoppler ist dazu ausgelegt, durch Kopplung mit einem zweiten Applikationskopfkoppler des Applikationskopfes bei bestehender Kopplung des ersten quellseitigen Zwischenelementkopplers mit dem ersten Applikationskopfkoppler das Zwischenelement starr an den Applikationskopf zu koppeln.

Bei einer derartigen Ausgestaltung des Zwischenelements wird das Zwischenelement in prinzipiell gleicher Weise an den Applikationskopf gekoppelt, wie das Patienteninterface an das Zwischenelement. Daher können für die Ausgestaltung des ersten und zweiten quellseitigen Zwischenelementkopplers die grundsätzlich gleichen Konstruktionen und Prinzipien angewandt werden, wie für den ersten und zweiten quellseitigen Patienteninterfacekoppler. Für beispielhafte Ausgestaltungen wird daher auf die obigen Ausführungen verwiesen, wobei bezüglich der Kopplung mit dem Applikationskopf das Zwischenelement an die Stelle des Patienteninterface tritt.

In einem beispielhaften aber nicht zwingenden Einsatzverfahren wird das isolierte Patienteninterface an das Patientenauge gekoppelt, beispielsweise, wie oben beschrieben, mittels Vakuum. Anschliessend wird das Patienteninterface durch die im Betriebszustand dem Applikationskopf zugewandte konkave Seite des Patienteninterface wie eine Schale teilweise mit Koppelflüssigkeit befüllt, wobei die Koppelflüssigkeit direkt in Kontakt mit der Hornhaut des Patientenauges gelangt. Separat davon wird das Zwischenelement an den Applikationskopf gekoppelt bzw. montiert. In einem letzten Schritt erfolgt die Kopplung des mit dem Patientenauge gekoppelten Patienteninterface mit dem Zwischenelement.

Der erste und/oder zweite quellseitige Zwischenelementkoppler und die korrespondierenden Gegenstücke des Applikationskopfes können, wie oben dargestellt, jeweils durch ein einzelnes Element oder durch zwei/oder mehrere Einzelelemente gebildet werden, wobei für die Anzahl und Anordnung die gleichen Überlegungen und Grundsätze gelten.

Gemäss einem weiteren Aspekt wird ein zur Verwendung in Verbindung mit dem Patienteninterface ausgelegter ophthalmologischer Applikationskopf zur Anwendung von optischer Strahlung einer Strahlquelle bereitgestellt.

Der ophthalmologische Applikationskopf umfasst einen ersten Applikationskopfkoppler und einen zweiten Applikationskopfkoppler. Der erste Applikationskopfkoppler und der zweite Applikationskopfkoppler sind dabei als erstes Patienteninterfacekoppler-Gegenstück zur Kopplung mit einem ersten quellseitigen Patienteninterfacekoppler und als zweites Patienteninterfacekoppler-Gegenstück zur Kopplung mit einem zusätzlichen zweiten quellseitigen Patienteninterfacekoppler des Patienteninterface ausgelegt. Alternativ sind der erste Applikationskopfkoppler und der zweite Applikationskopfkoppler zur Kopplung mit einem ersten quellseitigen Zwischenelementkoppler und einem zweiten quellseitigen Zwischenelementkoppler eines Zwischenelements ausgelegt. Der erste und zweite Applikationskopfkoppler können in beispielhaften Ausführungsformen so gestaltet sein, wie oben im Zusammenhang der Kopplung des Patienteninterface oder Zwischenelementes an den Applikationskopf erläutert wurde.

Gemäss einem weiteren Aspekt wird weiterhin ein Verfahren zur Kopplung eines ophthalmologischen Applikationskopfes zur Anwendung von optischer Strahlung einer Strahlquelle an ein Patientenauge bereitgestellt.

Das Verfahren zur Kopplung des ophthalmologischen Applikationskopfes mittels eines Patienteninterface an ein Patientenauge umfasst die folgenden sequentiellen Schritte:
- Herstellen eines Kontaktes des Patienteninterface mit dem Applikationskopf oder einem zur Anordnung zwischen Applikationskopf und Patienteninterface vorgesehenen Zwischenelement,
- Einschränken der Beweglichkeit des Patienteninterface bezüglich des Applikationskopfes oder des Zwischenelements durch Herstellen einer ersten Kopplung zwischen Patienteninterface und Applikationskopf oder Zwischenelement,
- starres Koppeln des Patienteninterface an den Applikationskopf oder das Zwischenelement durch Herstellen einer zweiten Kopplung zwischen Patienteninterface und Applikationskopf unter Beibehaltung der ersten Kopplung.

Das Verfahren kann ferner den Schritt der starren Kopplung eines Zwischenelementes and den Applikationskopf umfassen. Weitere optionale Verfahrensschritte ergeben sich unmittelbar aus der obigen Darstellung des Patienteninterface, Zwischenelements und Applikationskopfs, sowie nachfolgenden Ausführungsbeispielen.

Insbesondere bilden das Patienteninterface und das Zwischenelement ein ophthalmologisches Schutzsystem zur Anwendung mit dem Applikationskopf, welches zur Ausführung des angeführten Verfahrens geeignet ist. Offenbarte Ausführungsformen von Patienteninterface, Zwischenelement und Applikationskopf offenbaren daher zugleich entsprechende Ausgestaltungen eines entsprechenden Schutzsystems und Verfahrens. In gleicher Weise offenbaren beschriebene beispielhafte Verfahrensschritte zugleich entsprechende Ausgestaltungen von Patienteninterface, Zwischenelement und Applikationskopf respektive von einem dadurch gebildeten Schutzsystem.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
- Figuren 1 und 2:: zeigen einen Applikationskopf zur Anwendung von optischer Strahlung einer Strahlquelle zusammen mit einem Zwischenelement, einem Patienteninterface und einem Patientenauge.
- Figuren 3a und 3b:: zeigen schematisch ein Patienteninterface im Querschnitt zusammen mit einem Patientenauge.
- Figuren 4a, 4b, 4c, 4d, 4e:: verdeutlichen in schematischen Schnittdarstellungen die Kopplung eines Patienteninterface, eines Zwischenelements und Applikationskopfes.
- Figur 5:: zeigt einen Ausschnitt eines Zwischenelementes.

### Wege zur Ausführung der Erfindung

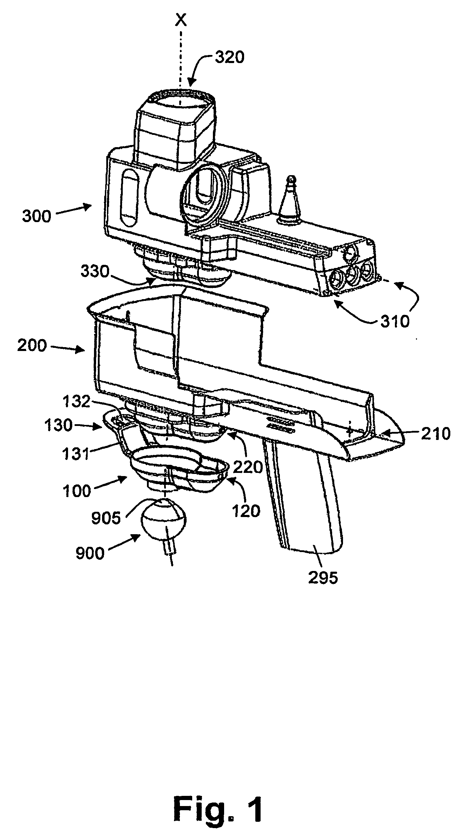

Die Figur 1 zeigt einen Applikationskopf 300 zur Anwendung von optischer Strahlung einer Strahlquelle, ein Zwischenelement 200 und ein Patienteninterface 100 in isometrischer Ansicht. Die Strahlquelle ist insbesondere eine Laserquelle zur Erzeugung von Laserpulsen respektive eines gepulsten Laserstrahls, beispielsweise zur Erzeugung von Femtosekundenlaserpulsen. Nachfolgend ist die Beschreibung beispielhaft auf Laserpulse ausgerichtet, doch der Fachmann wird verstehen, dass auch andere optische Strahlung und Strahlquellen verwendbar sind. Der Applikationskopf kann für therapeutische oder aber auch für diagnostische Zwecke eingesetzt werden. Es ist auch denkbar, dass nicht nur ein Applikationskopf an das Patienteninterface 100 gekoppelt wird, wie nachfolgend beschrieben wird, sondern mehrere, z.B. einer für chirurgische, ein anderer für diagnostische Zwecke. Es ist auch möglich, dass zunächst mit einem Laser therapiert wird, dann der Laser entfernt wird, das Patienteninterface 100 aber angedockt bleibt. Danach wird ein anderer chirurgischer Eingriff, wie zum Beispiel, die Entfernung des Inneren der Linse des Auges 900, durchgeführt und schliesslich wieder der Applikationskopf appliziert, um beispielsweise eine posteriore Capsulotomie des im Auge verbliebenen Kaspelsacks durchzuführen..

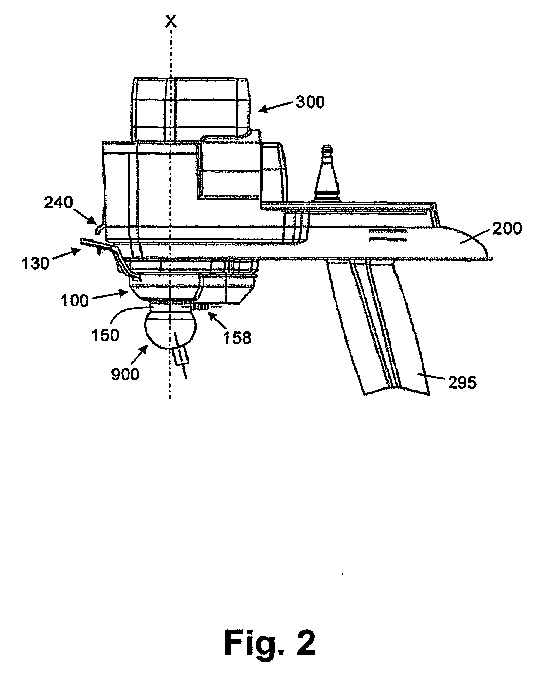

Figur 2 zeigt die gleichen Elemente im gekoppelten oder montierten Anwendungszustand in einer Seitenansicht. Die Elemente sind zur Verdeutlichung gemeinsam mit einem Patientenauge 900 mit Hornhaut 905 dargestellt. In der Figur 1 entspricht die relative Lage von Patienteninterface 100, Zwischenelement 200 und Applikationskopf 300 derjenigen von Figur 2, jedoch sind die Elemente durch einen Versatz entlang der optischen Projektionsachse X separiert.

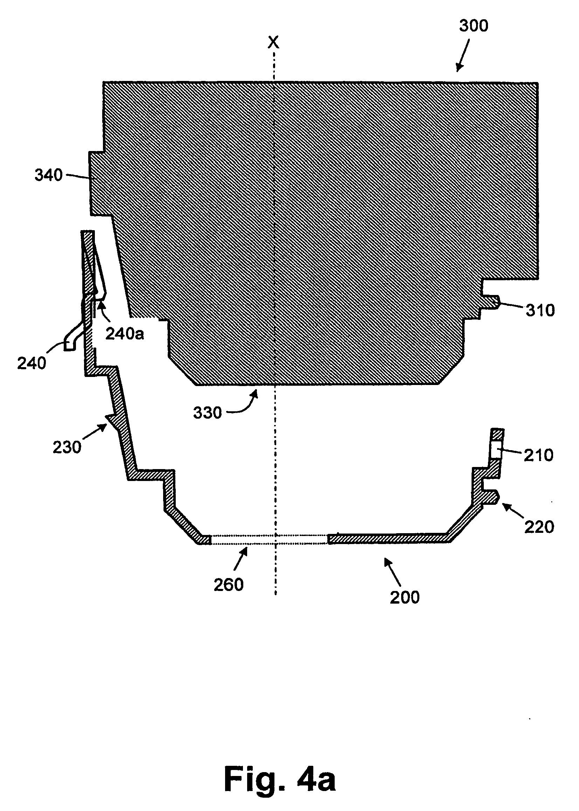

Der Applikationskopf 300 (zur Anwendung von optischer Strahlung einer Strahlquelle) hat ein Gehäuse mit einer im Wesentlichen konvexen Formgebung. An einer Stirnseite ragt von diesem der erste Applikationskopfkoppler ab, der beispielhaft durch zwei Pins 310 gebildet wird, wie in den Figuren 1 sowie 4a bis 4e ersichtlich ist.

Das Zwischenelement 200 hat eine schalenförmige Formgebung und weist in der dem Applikationskopf 300 zugewandten Seite eine im Wesentlichen konkave Formgebung auf, korrespondierend zur Form des Gehäuses des Applikationskopfes 300. Im montierten Zustand ist das Zwischenelement 200 wie eine Hülle über einen dem Patienten zugewandten Teil des Applikationskopfes 300 bzw. dessen Gehäuse gestülpt und umgibt diesen spielfrei. Exemplarisch hat das Zwischenelement 200 ferner einen im montierten Zustand vom Applikationskopf 300 wegweisenden Handgriff 295, wie in den Figuren 1 und 2 dargestellt ist. Das Zwischenelement 200 weist überdies ein Laser-Durchgangsfenster 260 auf. Das Laser-Durchgangsfenster 260 ist in einer Ausführungsvariante mit einer transparenten und flexiblen Schutzfolie abgedeckt, die beispielsweise an einem beweglichen Träger angebracht ist, der seinerseits umlaufend am Rand des Laser-Durchgangsfensters 260 befestigt ist.

Der zu den Pins 310 des ersten Applikationskopfkopplers korrespondierende erste quellseitige Zwischenelementkoppler ist beispielhaft durch zwei Bohrungen 210 realisiert, von denen in den Figuren 1 sowie 4a bis 4e jeweils nur eine sichtbar ist. Der bezüglich des ersten quellseitigen Zwischenelementkopplers an der gegenüberliegenden Stirnseite des Zwischenelements 200 angeordnete zweite quellseitige Zwischenelementkoppler ist als Federklinke 240 ausgebildet. Bei der Kopplung mit dem Applikationskopf 300 verrastet dieser mit einem (in Figur 1, Figur 2 nicht sichtbaren) Absatz 340 des Gehäuses vom Applikationskopf 300. Der Absatz 340 stellt entsprechend den zweiten Applikationskopfkoppler dar.

Das Patienteninterface 100 hat ebenso wie das Zwischenelement 200 eine schalenförmige Formgebung. Es weist an der dem Applikationskopf 300 und dem Zwischenelement 200 zugewandten Seite eine im Wesentlichen konkave Formgebung auf, korrespondierend zu der dem Patienten zugewandten konvexen Form des Zwischengehäuses 200. Im montierten Zustand ist das Patienteninterface 100 wie eine Hülle über einen dem Patienten zugewandten Teil des Zwischenelementes 200 gestülpt und starr mit diesem gekoppelt.

Das Patienteninterface 100 umfasst einen Saugring 150 mit kreisförmigem Querschnitt, dessen äusserer Durchmesser so bemessen ist, dass er vollständig auf der Sklera platziert werden kann. Seitlich ragt vom Saugring 150 ein Saugstutzen 158 zur Verbindung mit einer (nicht dargestellten) Vakuumpumpe ab.

Der erste quellseitige Patienteninterfacekoppler des Patienteninterface 100 wird beispielhaft durch eine Bohrung 120 in einer Wandung des Patienteninterface 100 gebildet, wie in den Figuren 1, 3a, 3b sowie 4b bis 4e dargestellt ist. Die Bohrung 120 korrespondiert mit einem vom Zwischenelement 200 abragenden Stift 220, welcher den ersten patientenseitigen Zwischenelementkoppler darstellt und in den Figuren 1 sowie 4a bis 4c illustriert ist.

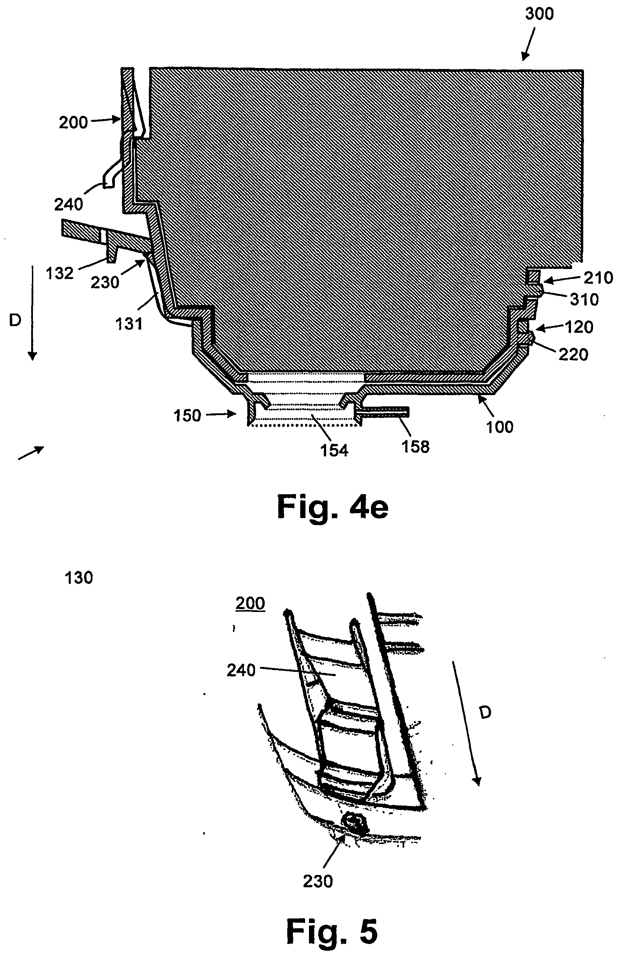

Der zweite quellseitige Patienteninterfacekoppler des Patienteninterface 100 wird beispielhaft durch eine federnde Klinke 130 gebildet. Der korrespondierende zweite patientenseitige Zwischenelementkoppler wird aus einer zur Klinke 130 korrespondierenden Gegenklinke 230 als Vorsprung am Zwischenelement 200 gebildet, welche in den Figuren 4a bis 4e und 5 dargestellt ist.

An dieser Stelle soll festgehalten werden, dass durch das in Figur 1 dargestellte Sichtfenster 320 einem Benutzer im Zustand des am Patientenauge 900 angedockten Systems, bestehend aus Patienteninterface 100, ggf. Zwischenelement 200 und Applikationskopf 300, eine Aufsicht auf das Patientenauge 900 ermöglicht wird, durch den Applikationskopf 300, das Laser-Austrittsfenster 330, das Laser-Durchgangsfenster 260 (ggf. einschliesslich Schutzfolie) des Zwischenelements 200 und den Saugring-Innenraum 154 des Patienteninterface 100 hindurch auf die Hornhaut 905 des Patientenauges 900.

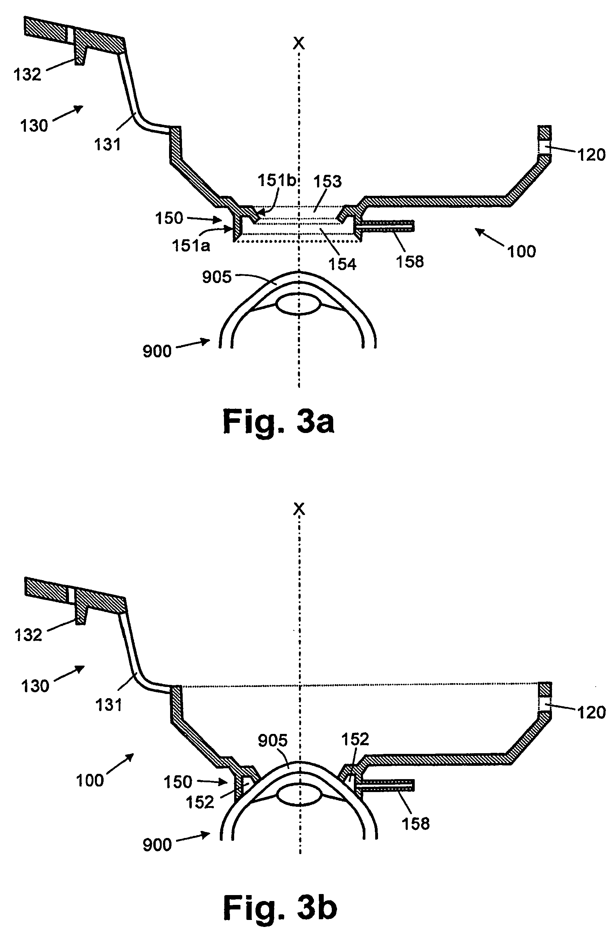

Nachfolgend wird spezifisch auf die Figuren 3a, 3b Bezug genommen. Figur 3a zeigt das Patienteninterface 100 schematisch in einer Querschnittszeichnung zusammen mit angedeutetem Patientenauge 900 im abgerückten Zustand, Figur 3b zeigt Patienteninterface 100 und Auge 900 im gekoppelten Zustand.

Der Saugring 150 umfasst in koaxialer Anordnung eine Saugring-Innenwand 151 b und eine Saugring-Aussenwand 151 a, die in ihrer Formgebung an die Wölbung des Auges 900 angepasst sind. Wie insbesondere aus Figur 3b ersichtlich, bilden Saugring-Aussenwand 151 a und die Saugring-Innenwand 151b gemeinsam eine ringförmige Hohlkammer 152, deren Stirnflächen auf der Hornhaut 905 aufliegen und in welche der Saugstutzen 158 mündet. Im in Figur 3b gezeigten Zustand kann mittels Saugstutzen 158 die Hohlkammer 152 evakuiert und in Unterdruck gebracht werden, wodurch das Patienteninterface 100 starr an das Auge 900 gekoppelt wird. Dabei ragt das Patientenauge 900, insbesondere ein Teil der Hornhaut 905, in den durch die SaugringInnenwand 151 b begrenzten Saugring-Innenraum 154 hinein.

Der erste quellseitige Patienteninterfacekoppler 130 umfasst zwei Federarme 131 und eine Entriegelungstaste 132, welche als mit der Hand greibare Griffstruktur gestaltet ist (siehe auch Figur 1).

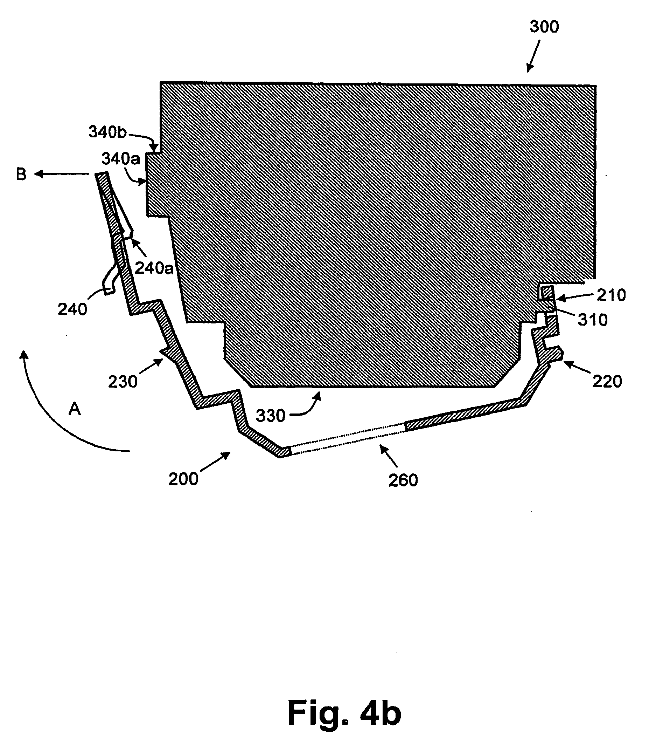

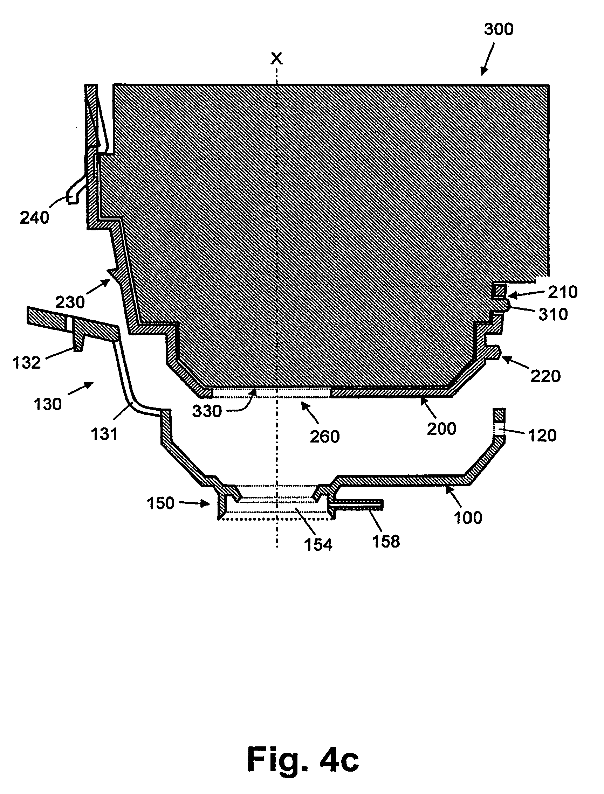

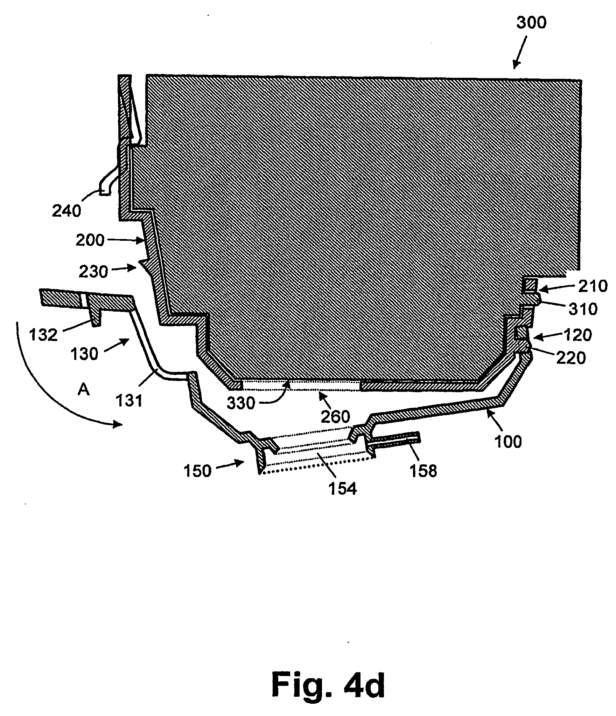

Nachfolgend wird explizit Bezug auf die Figuren 4a, 4b, 4c, 4d und 4e genommen. Die Figuren 4a, 4b und 4c verdeutlichen in einer schematischen Darstellung die Kopplung oder Montage des zunächst separaten (siehe Figur 4a) Zwischenelements 200 an den Applikationskopf 300. Zur Montage werden zunächst der erste Applikationskopfkoppler (Pin 310) mit dem ersten quellseitigen Zwischenelementkoppler (Bohrung 210) gekoppelt und so die Bewegungsfreiheit des Zwischenelements 200 bezüglich des Applikationskopfes 300 eingeschränkt. Dies geschieht im gezeigten Ausführungsbeispiel dadurch, dass die Pins 310 (erster Applikationskopfkoppler) in die Bohrungen 210 (erster quellseitiger Zwischenelementkoppler) eingehängt werden. In diesem Zustand, der in Fig. 4b dargestellt ist, ist das Zwischenelement 200 bezüglich des Applikationskopfes 300 geneigt bzw. verkippt.

Anschliessend wird das Zwischenelement 200 bezüglich des Applikationskopfes 300 in der durch Pfeil A angedeuteten Richtung gekippt bzw. geschwenkt (siehe Figur 4b), wobei die durch den Eingriff der Pins 310 in die Bohrungen 210 gebildete Kopplung bestehen bleibt. Im Zuge dieser Bewegung kommt die Kante 240a des Federhebels 240 in Kontakt mit der Interaktionsfläche 340a des Absatzes 340. Bei Fortsetzung der Kippoder Schwenkbewegung in Richtung A wird dadurch der Federhebel 240 federnd nach aussen, d. h. vom Applikationskopf 300 weg, gedrückt und gleitet bei der Bewegung über die Interaktionsfläche 340a. Sobald die Kante 240a über die Interaktionsfläche 340a und die Rastfläche 340b des Absatzes 340 gleitet, fällt die von der Interaktionsfläche 340a ausgeübte Kraft in Richtung B weg, und der Federhebel 240 schnappt entgegen Richtung B zurück in Richtung Applikationskopf 300 in eine Endstellung, in der der Federhebel 240 auf der Rastfläche 340b aufliegt. Dies entspricht der Endstellung des Zwischenelements 200, in der dieses starr an den Applikationskopf 300 gekoppelt bzw. montiert ist. Diese Konfiguration ist in Fig. 4c dargestellt. Es sind auch Lösungen möglich, die einzig auf Translation basieren.

Figur 5 zeigt in einer schematischen isometrischer Darstellung die Aussenansicht eines Abschnittes des Zwischenelements 200 mit Federklinke 240.

Ein späteres Trennen des Zwischenelements 200 vom Applikationskopf 300 kann einfach dadurch erfolgen, dass die Federklinke 240 federnd nach aussen, d. h. vom Applikationskopf 300 weg in Richtung B, gezogen wird, wodurch der Eingriff von Federklinke 240 und Rastfläche 340b aufgehoben wird.

Vorteilhaft sind die Federklinke 240 und der Absatz 340 so ausgelegt, dass die Federklinke 240 in der Endposition gemäss Figur 4c nicht vollständig entspannt ist, sondern nach innen (entgegen Richtung B) eine Federkraft auf den Applikationskopf 300 ausübt, wodurch eine definierter und spielfreier Sitz des Zwischenelements 200 auf dem Applikationskopf 300 gewährleistet ist. Weiterhin kann das gesamte Zwischenelement 200 im montierten Zustand von Fig. 3b geringfügig elastisch gedehnt sein. Ferner bilden die Aussenkontur des Applikationskopfes 300 und die Innenkontur der konkaven Form des Zwischenelementes 200 eine im Wesentlichen spielfreie Gleitpaarung. Das Gehäuse des Applikationskopfes 300 liegt also im Wesentlichen spielfrei an die innere Wandung des Zwischenelements 200 an. Aufgrund der bevorzugt vorhandenen Elastizität des Zwischenelements 200 kann das Gehäuse des Applikationskopfes 300 im entspannten Ausgangszustand auch ein geringfügiges Übermass über die Innenkontur des Zwischenelements 200 aufweisen.

In den Figuren 4c, 4d, 4e ist ferner die Montage respektive Kopplung des Patienteninterface 100 an das Zwischenelement 200 respektive an den Applikationskopf 300 dargestellt. Dabei zeigt Figur 4c das Patienteninterface in vom Zwischenelement 200 und Applikationskopf 300 abgerückter Stellung. Auch wenn dies aus Gründen der Klarheit und Übersichtlichkeit nicht dargestellt ist, kann das Patienteninterface 100 über den Saugring 150 bereits an das Patientenauge 900 gekoppelt sein, wie zuvor unter Bezug auf die Figuren 3a, 3b erläutert wurde. Alternativ kann das Patienteninterface 100 jedoch auch isoliert montiert werden.

Die Montage des Patienteninterface 100 an das Zwischenelement 200 erfolgt in grundsätzlich gleicher Weise wie die Montage des Zwischenelementes 200 an den Applikationskopf 300 und wird daher entsprechend knapp dargestellt.

Zur Montage werden zunächst der erste quellseitige Patienteninterfacekoppler (Bohrung 120) mit dem ersten patientenseitigen Zwischenelementkoppler (Pin 220) gekoppelt und so die Bewegungsfreiheit des Patienteninterface 100 bezüglich Zwischenelement 200 und Applikationskopf 300 eingeschränkt. Ebenso wie der erste Applikationskopfkoppler 310 ist der erste patientenseitige Zwischenelementkoppler 220 als Pin 220 ausgeführt; ebenso wie der erste quellseitige Zwischenelementkoppler 210 ist der erste quellseitige Patienteninterfacekoppler als Bohrung 120 ausgeführt. Aufgrund der im Vergleich zum Zwischenelement 200 geringeren Abmessungen des Patienteninterface 100 ist exemplarisch ein einziger Pin 220 und eine einzige Bohrung 120 in z. B. mittiger Anordnung vorgesehen. Der Zustand nach Einhängen des Pins 220 (erster patientenseitiger Zwischenelementkoppler) in Bohrung 120 (erster quellseitiger Patienteninterfacekoppler) ist in der Figur 4d argestellt.

Insbesondere wenn das Patienteninterface 100 bereits an das Patientenauge 900 gekoppelt und der Innenraum des Patienteninterface 100 ggf. teilweise mit Koppelflüssigkeit gefüllt ist (die Flüssigkeitsinjektion erfolgt ggf. nach dem Ankoppeln des Patienteninterface 100 an das Auge 900 und dem Koppeln an den Applikationskopf), erfolgt die erforderliche Relativbewegung in der Praxis bevorzugt nicht in erster Linie durch Bewegung des Patienteninterface 100, sondern durch Bewegung des Applikationskopfes 300 mit Zwischenelement 200, wobei das Patienteninterface 100 z.B. mittels seiner Griffstruktur unterstützt und gehalten wird. Das heisst, in Abweichung der schematischen Darstellung in Figur 4d, wird in der Praxis bei bereits am Auge 900 befestigten Zustand des Patienteninterface 100, der Applikationskopf 300 mit dem daran befestigten Zwischenelement 200 verkippt und durch Einhängen des Pins 220 in die Bohrung 120 an das Patienteninterface 100 eingehängt, ohne dass dabei das Patienteninterface 100 respektive das Auge 900 bewegt werden muss.

In einer Ausführungsvariante weist das Patienteninterface 100 einen PatienteninterfaceInnenraum 153 auf (siehe Figur 3a), der sich hin zum Patientenauge 900 konisch verjüngt, beispielsweise dadurch, dass der durch den inneren Saugringwand 151 b definierte Innenraum 153 sich im applizierten Zustand in Richtung des Patientenauges 900 zunehmend verengt. Der Patienteninterface-Innenraum 153 ist vorgesehen einen entsprechend konisch ausgestalteten transparenten Kontaktkörper (entfernbar) aufzunehmen (anstelle der Flüssigkeit). Der Kontaktkörper weist quellseitig eine ebene Fläche zum Applikationskopf 300 respektive dessen Laser-Austrittsfenster 330 hin auf (z.B. normal zur optischen Achse verlaufend). Der Kontaktkörper ist quellseitig an den Applikationskopf 300 ankoppelbar (z.B. an ein ebenes Laseraustrittsfenster). In einer Variante ist der Kontaktkörper an seiner dem Auge 900 zugewandten (verjüngten) Seite plan ausgestaltet und applaniert dadurch das Auge 900 respektive die Hornhaut 905 im applizierten Zustand. Es können auch verschiedene Patienteninterface 100 bereitgestellt werden, eines zum Applanieren mit einem Kontaktkörper, das andere für die Aufnahme von Flüssigkeit. Als Materialien für den Kontaktkörper können starre, elastische oder gelartige Materialen eingesetzt werden.

Anschliessend wird das Patienteninterface 100 bezüglich des Applikationskopfes 300 und des Zwischenelementes 200 in der durch Pfeil A angedeuteten Richtung gekippt bzw. geschwenkt, wobei der Eingriff von Pin 220 und Bohrungen 120 bestehen bleibt. Ebenso wie bei der vorherigen Bewegung kann das Patienteninterface 100 gehalten und stattdessen der Applikationskopf 300 mit dem Zwischenelement 200 gekippt bzw. geschwenkt werden.

In der Endposition verrastet die federnde Klinke 130 als zweites quellseitiger Patienteninterfacekoppler mit der Gegenklinke 230 (siehe auch Figur 5) als zweiter patientenseitiger Zwischenelementkoppler, wie in der Figur 4e dargestellt ist. Die Gegenklinke 230 wirkt dabei mit der federnden Klinke 130 in gleicher Weise zusammen, wie die Federklinke 240 mit dem Absatz 340, wobei ersichtlich ist, dass die jeweils konkret verwendete Ausführung für die Funktion unerheblich ist und entsprechend lediglich Ausführungsbeispiele darstellen.

Ein späteres Trennen des Patienteninterface 100 vom Zwischenelement 200 kann einfach dadurch erfolgen, dass die Entriegelungstaste 132 in Richtung C gedrückt wird, d. h. vom Applikationskopf 300 weg. Hierdurch wird der Eingriff von Federklinke 130 und Gegenklinke 230 aufgehoben.

Da die Montage bzw. Kopplung des Zwischenelementes 200 an den Applikationskopf 300 sowie die Montage bzw. Kopplung des Patienteninterface 100 an das Zwischenelement 200 auf analoge Weise und nach den gleichen Prinzipien erfolgen, ist ferner ersichtlich, das bei entsprechender konstruktiver Ausgestaltung von Patienteninterface 100 und Applikationskopf 300 auch eine direkte Kopplung dieser Elemente ohne das Zwischenelement 200 auf die gleiche Art und Weise möglich ist.

Das Zwischenelement 200 und das Patienteninterface 100 sind in einer Variante jeweils einstückig ausgeführt und in Spritzgusstechnik aus Kunststoff hergestellt, beispielsweise aus einem Polycarbonat. Das Patienteninterface ist mindestens teilweise elastisch ausgeführt und wird beim Koppeln des zweiten quellseitigen Patienteninterfacekopplers 130 mit dem zweiten Patienteninterfacekoppler-Gegenstück 230 federnd gespannt.

### Liste der Bezugszeichen

- 100: Patienteninterface
- 120: Bohrung / erster quellseitiger Patienteninterfacekoppler
- 130: Federklinke / zweiter quellseitiger Patienteninterfacekoppler
- 131: Federarm
- 132: Entriegelungstaste
- 150: Saugring
- 151 a: äussere Saugringwand
- 151b: innere Saugringwand
- 152: Zwischenraum
- 153: Patienteninterface-Innenraum
- 154: Saugring-Innenraum
- 158: Saugstutzen
- 200: Zwischenelement
- 210: Bohrung / erster quellseitiger Zwischenelementkoppler
- 220: Pin / erster patientenseitiger Zwischenelementkoppler
- 230: Gegenklinke / zweiter patientenseitiger Zwischenelementkoppler
- 240: Federklinke / zweiter quellseitiger Zwischenelementkoppler
- 240a: Rastfläche
- 260: Laser-Durchgangsfenster / Schutzfolie
- 295: Handgriff
- 300: Applikationskopf zur Anwendung von optischer Strahlung einer Strahlquelle
- 310: Pin / erster Applikationskopfkoppler
- 320: Sichtfenster
- 330: Laser-Austrittsfenster
- 340: Absatz / zweiter Applikationskopfkoppler
- 340a: Interaktionsfläche
- 340b: Rastfläche
- 900: Patientenauge
- 905: Hornhaut

## Patentansprüche

1. Patienteninterface (100) zur Kopplung eines ophthalmologischen Applikationskopfes (300) zur Anwendung von optischer Strahlung einer Strahlquelle an ein Patientenauge (900), das Patienteninterface (100) umfassend:
eine patientenseitige Interface-Struktur (150), die zur Kopplung an das Patientenauge (900) ausgestaltet ist;
eine quellseitige Interfacestruktur, welche dazu ausgelegt ist, das Patienteninterface (100) starr an den Applikationskopf (300) oder an ein zur Anordnung zwischen Applikationskopf (300) und Patienteninterface (100) vorgesehenes Zwischenelement (200) zu koppeln,
wobei die quellseitige Interfacestruktur einen ersten quellseitigen Patienteninterfacekoppler (120) und einen zusätzlichen zweiten quellseitigen Patienteninterfacekoppler (130) umfasst,
**gekennzeichnet dadurch, dass**:
der erste quellseitige Patienteninterfacekoppler (120) dazu ausgelegt ist, ausgehend von einem ungekoppelten Ausgangszustand, in dem das Patienteninterface (100) nicht mit dem Applikationskopf (300) bzw. dem ggf. vorhandenen Zwischenelement (200) gekoppelt und von diesem separat ist - durch Herstellen einer Kopplung mit einem ersten Patienteninterfacekoppler-Gegenstück (220) des Applikationskopfes (300) oder des Zwischenelements (200) die Beweglichkeit des Patienteninterface (100) bezüglich des Applikationskopfes (300) oder des Zwischenelements (200) einzuschränken, und
der zweite quellseitige Patienteninterfacekoppler (130 dazu ausgelegt ist, durch Herstellen einer Kopplung mit einem zweiten Patienteninterfacekoppler-Gegenstück (230) des Applikationskopfes (300) oder des Zwischenelements (200) das Patienteninterface (100) bei bestehender Kopplung des ersten quellseitigen Patienteninterfacekopplers (120) mit dem ersten Patienteninterfacekoppler-Gegenstück (220) starr an den Applikationskopf (300) oder das Zwischenelement (200) zu koppeln.

2. Patienteninterface (100) nach Anspruch 1, **dadurch gekennzeichnet, dass** der erste quellseitige Patienteninterfacekoppler (120) dazu ausgelegt ist, unter Beibehaltung mindestens eines Freiheitsgrades mit dem ersten Patienteninterfacekoppler-Gegenstück (220) formschlüssig zu koppeln.

3. Patienteninterface (100) nach Anspruch 1 oder 2, **dadurch gekennzeichnet, dass** das Herstellen einer Kopplung des zweiten quellseitigen Patienteninterfacekopplers (130) mit dem zweiten Patienteninterfacekoppler-Gegenstück (230) erfordert, dass der erste quellseitige Patienteninterfacekoppler (1 20) mit dem ersten Patienteninterfacekoppler-Gegenstück (220) gekoppelt ist.

4. Patienteninterface (100) nach Anspruch 3, **dadurch gekennzeichnet, dass** der erste quellseitige Patienteninterfacekoppler (120) dazu ausgelegt ist, durch Herstellen einer Kopplung mit dem ersten Patienteninterfacekoppler-Gegenstück (220) eine Zwangsführung für das Patienteninterface (100) bezüglich des Applikationskopfes (300) oder des Zwischenelements (200) herzustellen.

5. Patienteninterface (100) nach einem der Ansprüche 1 bis 4, **dadurch gekennzeichnet, dass** der zweite quellseitige Patienteninterfacekoppler (130) dazu ausgelegt ist, mit dem zweiten Patienteninterfacekoppler-Gegenstück (230) mittels Schnapp- oder Rastverbindung zu koppeln.

6. Patienteninterface (100) nach einem der Ansprüche 1 bis 5, **dadurch gekennzeichnet, dass** das Patienteninterface (100) mindestens teilweise elastisch ist und beim Herstellen der Kopplung des zweiten quellseitigen Patienteninterfacekopplers (130) mit dem zweiten Patienteninterfacekoppler-Gegenstück (230) federnd gespannt wird.

7. Patienteninterface (100) nach einem der Ansprüche 1 bis 6, **dadurch gekennzeichnet, dass** das Herstellen der Kopplung des ersten quellseitigen Patienteninterfacekopplers (120) mit dem ersten Patienteninterfacekoppler-Gegenstück (220) erfordert, dass der zweite quellseitige Patienteninterfacekoppler (130) mit dem zweiten Patienteninterfacekoppler-Gegenstück (230) nicht gekoppelt ist.

8. Patienteninterface (100) nach einem Ansprüche 1 bis 7, **dadurch gekennzeichnet, dass** das Patienteninterface (100) eine mit einer Hand haltbare Griffstruktur umfasst.

9. Patenteninterface (100) nach Anspruch 8, **dadurch gekennzeichnet, dass** die Griffstruktur integral mit dem zweiten quellseitigen Patienteninterfacekoppler (130) ist.

10. Patienteninterface (100) nach einem der Ansprüche 1 bis 9, **dadurch gekennzeichnet, dass** die patientenseitige Interfacestruktur einen Saugring (150) umfasst, welcher zum Herstellen einer starren Vakuumkopplung mit dem Patientenauge (900) ausgebildet ist.

11. Patienteninterface (100) nach einem der Ansprüche 1 bis 10, **dadurch gekennzeichnet, dass** der zweite quellseitige Patienteninterfacekoppler (130) einen einhändig lösbaren Schnapphebel umfasst.

12. Zwischenelement (200) zur Anordnung zwischen einem ophthalmologischen Applikationskopf (300) zur Anwendung von optischer Strahlung einer Strahlquelle und einem Patienteninterface (100) nach einem der Ansprüche 1 bis 11, das Zwischenelement (200) umfassend:
- eine patientenseitige Zwischenelement -Interfacestruktur;
- eine quellseitige Zwischenelement-Interfacestruktur,
wobei die patientenseitige Zwischenelement-Interfacestruktur einen ersten patientenseitigen Zwischenelementkoppler (220) und einen zusätzlichen zweiten patientenseitigen Zwischenelementkoppler umfasst (230),
wobei der erste patientenseitige Zwischenelementkoppler (220) ein erstes Patienteninterfacekoppler-Gegenstück bildet und der zweite patientenseitige Zwischenelementkoppler (230) ein zweites Patienteninterfacekoppler-Gegenstück bildet,
wobei der erste patientenseitige Zwischenelementkoppler (220) dazu ausgelegt ist, durch Herstellen einer Kopplung mit dem ersten quellseitigen Patienteninterfacekoppler (120) des Patienteninterface (100) die Beweglichkeit des Patienteninterface (100) bezüglich des Zwischenelements (200) einzuschränken,
und wobei der zweite patientenseitige Zwischenelementkoppler (230) dazu ausgelegt ist, durch Herstellen einer Kopplung mit dem zweiten quellseitigen Patienteninterfacekoppler (130) des Patienteninterface (100) bei bestehender Kopplung des ersten patientenseitigen Zwischenelementkopplers (220) mit dem ersten quellseitigen Patienteninterfacekoppler (120) das Patienteninterface (100) starr an das Zwischenelement (200) zu koppeln.

13. Zwischenelement (200) nach Anspruch 12, **dadurch gekennzeichnet, dass** die quellseitige Zwischenelement-Interfacestruktur dazu ausgelegt ist, das Zwischenelement (200) starr an den Applikationskopf (300) zu koppeln, dass die quellseitige Zwischenelement-Interfacestruktur einen ersten quellseitigen Zwischenelementkoppler (210) und einen zweiten quellseitigen Zwischenelementkoppler (240) umfasst, wobei der erste quellseitige Zwischenelementkoppler (210) dazu ausgelegt ist, durch Herstellen einer Kopplung mit einem ersten Applikationskopfkoppler (310) des Applikationskopfes (300) die Beweglichkeit des Zwischenelements (200) bezüglich des Applikationskopfes (300) einzuschränken und der zweite quellseitige Zwischenelementkoppler (240) dazu ausgelegt ist, durch Kopplung mit einem zweiten Applikationskopfkoppler (340) des Applikationskopfes (300) bei bestehender Kopplung des ersten quellseitigen Zwischenelementkopplers (210) mit dem ersten Applikationskopfkoppler (310) das Zwischenelement (200) starr an den Applikationskopf (300) zu koppeln.

14. Ophthalmologischer Applikationskopf (300) zur Anwendung von optischer Strahlung einer Strahlquelle umfassend einen ersten Applikationskopfkoppler (310) und einen zweiten Applikationskopfkoppler (320), wobei der erste Applikationskopfkoppler (310) und der zweite Applikationskopfkoppler (320)
- als erstes Patienteninterfacekoppler-Gegenstück zur Kopplung mit einem ersten quellseitigen Patienteninterfacekoppler (120) und als zweites Patienteninterfacekoppler-Gegenstück zur Kopplung mit einem zusätzlichen zweiten quellseitigen Patienteninterfacekoppler (130) eines Patienteninterface nach einem der Ansprüche 1-10, oder
- zur Kopplung mit einem ersten quellseitigen Zwischenelementkoppler (210) und einem zweiten quellseitigen Zwischenelementkoppler (240) eines Zwischenelements (200) nach einem der Ansprüche 12 bis 13 ausgelegt sind.

15. Verfahren zur Kopplung eines ophthalmologischen Applikationskopfes (300) zur Anwendung von optischer Strahlung einer Strahlquelle mittels eines Patienteninterface (100) an ein Patientenauge (900), das Verfahren umfassend die folgenden sequentiellen Schritte:
- Ausgehend von einem ungekoppelten Ausgangszustand, in dem das Patienteninterface (100) nicht mit dem Applikationskopf (300) bzw. einem ggf. vorhandenen Zwischenelement (200) gekoppelt und von diesem separat ist: Herstellen eines Kontaktes des Patienteninterface (100) mit dem Applikationskopf (300) oder einem zur Anordnung zwischen Applikationskopf (300) und Patienteninterface (100) vorgesehenen Zwischenelement (200),
- Einschränken der Beweglichkeit des Patienteninterface (100) bezüglich des Applikationskopfes (300) oder des Zwischenelements (200) durch Herstellen einer ersten Kopplung zwischen dem Patienteninterface (100) und dem Applikationskopf (300) oder Zwischenelement (200),
- starres Koppeln des Patienteninterface (100) an den Applikationskopf (300) oder das Zwischenelement (200) durch Herstellen einer zweiten Kopplung zwischen Patienteninterface (100) und Applikationskopf (300) unter Beibehaltung der ersten Kopplung.

## Claims

1. Patient interface (100) for coupling an ophthalmological application head (300) for applying optical radiation of a radiation source onto an eye (900) of a patient, the patient interface (100) comprising:
a patient-side interface structure (150), which is designed for coupling to the eye (900) of a patient;
a source-side interface structure, which is designed to couple the patient interface (100) rigidly to the application head (300) or to an intermediate element (200) provided to be arranged between application head (300) and patient interface (100),
wherein the source-side interface structure comprises a first source-side patient interface coupler (120) and an additional second source-side patient interface coupler (130), **characterized in that**:
the first source-side patient interface coupler (120) is designed such that, proceeding from an uncoupled starting state in which the patient interface (100) is not coupled to the application head (300) or the optionally present intermediate element (200) and is thus separate therefrom, it limits the mobility of the patient interface (100) with respect to the application head (300) or the intermediate element (200) by producing a coupling to a first patient interface coupler counterpiece (220) of the application head (300) or of the intermediate element (200), and
the second source-side patient interface coupler (130) is designed such that, by producing a coupling to a second patient interface coupler counterpiece (230) of the application head (300) or of the intermediate element (200), it couples the patient interface (100) rigidly to the application head (300) or the intermediate element (200) while the first source-side patient interface coupler (120) is already coupled to the first patient interface coupler counterpiece (220).

2. Patient interface (100) according to Claim 1, **characterized in that** the first source-side patient interface coupler (120) is designed to couple with form-fit engagement to the first patient interface coupler counterpiece (220) while maintaining at least one degree of freedom.

3. Patient interface (100) according to Claim 1 or 2, **characterized in that**, in order to produce a coupling of the second source-side patient interface coupler (130) to the second patient interface coupler counterpiece (230), it is necessary that the first source-side patient interface coupler (120) is coupled to the first patient interface coupler counterpiece (220).

4. Patient interface (100) according to Claim 3, **characterized in that** the first source-side patient interface coupler (120) is designed such that, by producing a coupling to the first patient interface coupler counterpiece (220), it produces a constrained guide for the patient interface (100) with respect to the application head (300) or the intermediate element (200).

5. Patient interface (100) according to one of Claims 1 to 4, **characterized in that** the second source-side patient interface coupler (130) is designed to couple to the second patient interface coupler counterpiece (230) by means of a snap-fitting connection or latching connection.

6. Patient interface (100) according to one of Claims 1 to 5, **characterized in that** the patient interface (100) is at least partially elastic and, upon production of the coupling of the second source-side patient interface coupler (130) to the second patient interface coupler counterpiece (230), is elastically tensioned.

7. Patient interface (100) according to one of Claims 1 to 6, **characterized in that**, in order to produce the coupling of the first source-side patient interface coupler (120) to the first patient interface coupler counterpiece (220), it is necessary that the second source-side patient interface coupler (130) is not coupled to the second patient interface coupler counterpiece (230).

8. Patient interface (100) according to one of Claims 1 to 7, **characterized in that** the patient interface (100) comprises a grip structure that can be held with one hand.

9. Patient interface (100) according to Claim 8, **characterized in that** the grip structure is integral with the second source-side patient interface coupler (130).

10. Patient interface (100) according to one of Claims 1 to 9, **characterized in that** the patient-side interface structure comprises a suction ring (150), which is designed to produce a rigid vacuum coupling to the eye (900) of a patient.

11. Patient interface (100) according to one of Claims 1 to 10, **characterized in that** the second source-side patient interface coupler (130) comprises a snap lever that is releasable with one hand.

12. Intermediate element (200) for arrangement between an ophthalmological application head (300), for applying optical radiation of a radiation source, and a patient interface (100) according to one of Claims 1 to 11, said intermediate element (200) comprising:
- a patient-side intermediate element interface structure;
- a source-side intermediate element interface structure,
wherein the patient-side intermediate element interface structure comprises a first patient-side intermediate element coupler (220) and an additional second patient-side intermediate element coupler (230),
wherein the first patient-side intermediate element coupler (220) forms a first patient interface coupler counterpiece, and the second patient-side intermediate element coupler (230) forms a second patient interface coupler counterpiece,
wherein the first patient-side intermediate element coupler (220) is designed to limit the mobility of the patient interface (100) with respect to the intermediate element (200) by producing a coupling to the first source-side patient interface coupler (120) of the patient interface (100),
and wherein the second patient-side intermediate element coupler (230) is designed such that, by producing a coupling to the second source-side patient interface coupler (130) of the patient interface (100), it couples the patient interface (100) rigidly to the intermediate element (200) while the first patient-side intermediate element coupler (220) is already coupled to the first source-side patient interface coupler (120).

13. Intermediate element (200) according to Claim 12, **characterized in that** the source-side intermediate element interface structure is designed to couple the intermediate element (200) rigidly to the application head (300), **in that** the source-side intermediate element interface structure comprises a first source-side intermediate element coupler (210) and a second source-side intermediate element coupler (240), wherein the first source-side intermediate element coupler (210) is designed to limit the mobility of the intermediate element (200) with respect to the application head (300) by producing a coupling to a first application head coupler (310) of the application head (300), and the second source-side intermediate element coupler (240) is designed to couple the intermediate element (200) rigidly to the application head (300) by coupling to a second application head coupler (340) of the application head (300) while the first source-side intermediate element coupler (210) is already coupled to the first application head coupler (310).

14. Ophthalmological application head (300) for applying optical radiation of a radiation source, comprising a first application head coupler (310) and a second application head coupler (320), wherein the first application head coupler (310) and the second application head coupler (320) are designed
- as a first patient interface coupler counterpiece for coupling to a first source-side patient interface coupler (120), and as a second patient interface coupler counterpiece for coupling to an additional second source-side patient interface coupler (130) of a patient interface according to one of Claims 1-10, or
- for coupling to a first source-side intermediate element coupler (210) and a second source-side intermediate element coupler (240) of an intermediate element (200) according to one of Claims 12 to 13.

15. Method for coupling an ophthalmological application head (300) for applying optical radiation of a radiation source onto an eye (900) of a patient by means of a patient interface (100), said method comprising the following sequential steps:
- producing contact of the patient interface (100) to the application head (300) or to an intermediate element (200) provided for arrangement between application head (300) and patient interface (100), and doing this proceeding from an uncoupled starting state in which the patient interface (100) is not coupled to the application head (300) or to an optionally present intermediate element (200) and is thus separate therefrom,
- limiting the mobility of the patient interface (100) with respect to the application head (300) or the intermediate element (200) by producing a first coupling between the patient interface (100) and the application head (300) or intermediate element (200),
- rigidly coupling the patient interface (100) to the application head (300) or the intermediate element (200) by producing a second coupling between patient interface (100) and application head (300) while maintaining the first coupling.

## Revendications

1. Interface de patient (100) destinée à coupler une tête d'application ophtalmologique (300) en vue d'appliquer un rayonnement optique d'une source de rayonnement à l'oeil d'un patient (900), l'interface de patient (100) comprenant :
une structure d'interface côté patient (150) qui est configurée pour être couplée à l'oeil du patient (900) ;
une structure d'interface côté source qui est conçue pour coupler l'interface de patient (100) de manière rigide à la tête d'application (300) ou à un élément intermédiaire (200) prévu pour être disposé entre la tête d'application (300) et l'interface de patient (100),
la structure d'interface côté source comprenant un premier coupleur d'interface de patient côté source (120) et un deuxième coupleur d'interface de patient côté source (130) supplémentaire,
caractériséee en ce que
le premier coupleur d'interface de patient côté source (120) est conçu pour, en partant d'un état initial non couplé dans lequel l'interface de patient (100) n'est pas couplée à la tête d'application (300) ou à l'élément intermédiaire (200) éventuellement présent et
est séparée de celle-ci/celui-ci, restreindre la mobilité de l'interface de patient (100) par rapport à la tête d'application (300) ou à l'élément intermédiaire (200) en établissant un couplage avec une première pièce homologue de coupleur d'interface de patient (220) de la tête d'application (300) ou de l'élément intermédiaire (200), et
le deuxième coupleur d'interface de patient côté source (130) est conçu pour, en établissant un couplage avec une deuxième pièce homologue de coupleur d'interface de patient (230) de la tête d'application (300) ou de l'élément intermédiaire (200), coupler de manière rigide l'interface de patient (100) à la tête d'application (300) ou à l'élément intermédiaire (200) en présence d'un couplage existant du premier coupleur d'interface de patient côté source (120) avec la première pièce homologue de coupleur d'interface de patient (220).

2. Interface de patient (100) selon la revendication 1, **caractérisée en ce que** le premier coupleur d'interface de patient côté source (120) est conçu pour se coupler avec la première pièce homologue de coupleur d'interface de patient (220) par complémentarité de formes en conservant au moins un degré de liberté.

3. Interface de patient (100) selon la revendication 1 ou 2, **caractérisée en ce que** l'établissement d'un couplage du deuxième coupleur d'interface de patient côté source (130) avec la deuxième pièce homologue de coupleur d'interface de patient (230) impose que le premier coupleur d'interface de patient côté source (120) soit couplé avec la première pièce homologue de coupleur d'interface de patient (220).

4. Interface de patient (100) selon la revendication 3, **caractérisée en ce que** le premier coupleur d'interface de patient côté source (120) est conçu pour créer un guidage forcé pour l'interface de patient (100) par rapport à la tête d'application (300) ou à l'élément intermédiaire (200) en établissant un couplage avec la première pièce homologue de coupleur d'interface de patient (220).

5. Interface de patient (100) selon l'une des revendications 1 à 4, **caractérisée en ce que** le deuxième coupleur d'interface de patient côté source (130) est conçu pour se coupler avec la deuxième pièce homologue de coupleur d'interface de patient (230) au moyen d'une liaison par déclic ou par enclenchement.

6. Interface de patient (100) selon l'une des revendications 1 à 5, **caractérisée en ce que** l'interface de patient (100) est au moins partiellement élastique et serrée par ressort lors de l'établissement du couplage du deuxième coupleur d'interface de patient côté source (130) avec la deuxième pièce homologue de coupleur d'interface de patient (230).

7. Interface de patient (100) selon l'une des revendications 1 à 6, **caractérisée en ce que** l'établissement du couplage du premier coupleur d'interface de patient côté source (120) avec la première pièce homologue de coupleur d'interface de patient (220) impose que le deuxième coupleur d'interface de patient côté source (130) ne soit pas couplé avec la deuxième pièce homologue de coupleur d'interface de patient (230).

8. Interface de patient (100) selon l'une des revendications 1 à 7, **caractérisée en ce que** l'interface de patient (100) comprend une structure de poignée pouvant être tenue d'une main.

9. Interface de patient (100) selon la revendication 8, **caractérisée en ce que** la structure de poignée fait partie intégrante du deuxième coupleur d'interface de patient côté source (130).

10. Interface de patient (100) selon l'une des revendications 1 à 9, **caractérisée en ce que** la structure d'interface côté patient comprend une ventouse (150) qui est configurée pour établir un couplage sous vide rigide avec l'oeil du patient (900).

11. Interface de patient (100) selon l'une des revendications 1 à 10, **caractérisée en ce que** le deuxième coupleur d'interface de patient côté source (130) comprend un levier à déclic pouvant être libéré d'une main.

12. Élément intermédiaire (200) destiné à être disposé entre une tête d'application ophtalmologique (300) en vue d'appliquer un rayonnement optique d'une source de rayonnement et une interface de patient (100) selon l'une des revendications 1 à 11, l'élément intermédiaire (200) comprenant :
- une structure d'interface d'élément intermédiaire côté patient ;
- une structure d'interface d'élément intermédiaire côté source,
la structure d'interface d'élément intermédiaire côté patient comprenant un premier coupleur d'élément intermédiaire côté patient (220) et un deuxième coupleur d'élément intermédiaire côté patient (230) supplémentaire,
le premier coupleur d'élément intermédiaire côté patient (220) formant une première pièce homologue de coupleur d'interface de patient et le deuxième coupleur d'élément intermédiaire côté patient (230) formant une deuxième pièce homologue de coupleur d'interface de patient,
le premier coupleur d'élément intermédiaire côté patient (220) étant conçu pour, en établissant un couplage avec le premier coupleur d'interface de patient côté source (120) de l'interface de patient (100), restreindre la mobilité de l'interface de patient (100) par rapport à l'élément intermédiaire (200),
et le deuxième coupleur d'interface d'élément intermédiaire côté patient (230) étant conçu pour, en établissant un couplage avec le deuxième coupleur d'interface de patient côté source (130) de l'interface de patient (100), coupler de manière rigide l'interface de patient (100) à l'élément intermédiaire (200) en présence d'un couplage existant du premier coupleur d'interface d'élément intermédiaire côté patient (220) avec le premier coupleur d'interface de patient côté source (120).

13. Élément intermédiaire (200) selon la revendication 12, **caractérisée en ce que** la structure d'interface d'élément intermédiaire côté source est conçue pour coupler de manière rigide l'élément intermédiaire (200) à la tête d'application (300), **en ce que** la structure d'interface d'élément intermédiaire côté source comprend un premier coupleur d'élément intermédiaire côté source (210) et un deuxième coupleur d'élément intermédiaire côté source (240), le premier coupleur d'élément intermédiaire côté source (210) étant conçu pour, en établissant un couplage avec un premier coupleur de tête d'application (310) de la tête d'application (300), restreindre la mobilité de l'élément intermédiaire (200) par rapport à la tête d'application (300) et le deuxième coupleur d'élément intermédiaire côté source (240) étant conçu pour, par couplage avec un deuxième coupleur de tête d'application (340) de la tête d'application (300), coupler de manière rigide l'élément intermédiaire (200) à la tête d'application (300) en présence d'un couplage existant du premier coupleur d'élément intermédiaire côté source (210) avec le premier coupleur de tête d'application (310).

14. Tête d'application ophtalmologique (300) destinée à appliquer un rayonnement optique d'une source de rayonnement, comprenant un premier coupleur de tête d'application (310) et un deuxième coupleur de tête d'application (320), le premier coupleur de tête d'application (310) et le deuxième coupleur de tête d'application (320) étant conçus
- sous la forme d'une première pièce homologue de coupleur d'interface de patient destinée au couplage avec un premier coupleur d'interface de patient côté source (120) et sous la forme d'une deuxième pièce homologue de coupleur d'interface de patient destinée au couplage avec un deuxième coupleur d'interface de patient côté source (130) supplémentaire d'une interface de patient selon l'une des revendications 1 à 10, ou
- pour le couplage avec un premier coupleur d'élément intermédiaire côté source (210) et un deuxième coupleur d'élément intermédiaire côté source (240) d'un élément intermédiaire (200) selon l'une des revendications 12 à 13.

15. Procédé de couplage d'une tête d'application ophtalmologique (300) en vue d'appliquer un rayonnement optique d'une source de rayonnement à l'oeil d'un patient (900) au moyen d'une interface de patient (100), le procédé comprenant les étapes séquentielles suivantes :
- en partant d'un état initial non couplé dans lequel l'interface de patient (100) n'est pas couplée à la tête d'application (300) ou à un élément intermédiaire (200) éventuellement présent et est séparée de celle-ci/celui-ci : établissement d'un contact de l'interface de patient (100) avec la tête d'application (300) ou un élément intermédiaire (200) prévu pour être disposé entre la tête d'application (300) et l'interface de patient (100),
- restriction de la mobilité de l'interface de patient (100) par rapport à la tête d'application (300) ou à l'élément intermédiaire (200) en établissant un premier couplage entre l'interface de patient (100) et la tête d'application (300) ou l'élément intermédiaire (200),
- couplage rigide de l'interface de patient (100) à la tête d'application (300) ou à l'élément intermédiaire (200) en établissant un deuxième couplage entre l'interface de patient (100) et la tête d'application (300) en conservant le premier couplage.
